# EUROPEAN PATENT APPLICATION

(11) **EP 3 772 355 A1**
(43) Date of publication of application: **10.02.2021**
(21) Application number: 19382685.6
(22) Date of filing: 05.08.2019
(51) Int. Cl.: A61K 47/54, A61K 47/55

(54) **BIFUNCTIONAL COMPOUND AND ITS USE IN IMMUNOTHERAPY**

(71) Applicant: Consejo Superior de Investigaciones Científicas (CSIC), 28006 Madrid (ES)
(72) Inventor: MIRANDA CHINEA, Pedro Oswaldo, 38206 San Cristóbal de La Laguna (Santa Cruz de Tenerife) (ES); PADRÓN PEÑA, Juan Ignacio, 38206 San Cristóbal de La Laguna (Santa Cruz de Tenerife) (ES)
(74) Representative: Pons

(57) **Abstract**

The invention relates to a bifunctional compound that is, on one side, an agonist of the TLR4 and, on the other side, an important inhibitor of the PSMA. Said compound is useful in immunotherapy for the treatment and/or prevention of prostate cancer. Therefore, the invention also relates to the use of the compound and to the pharmaceutical composition comprising it.

## Description

The invention relates to a bifunctional compound and its use in immunotherapy. In particular, the compound is useful in immunotherapy for the treatment and/or prevention of prostate cancer. Therefore, the present invention belongs to the field of medicine and pharmacy.

### STATE OF ART

Prostate cancer is the second most common cancer worldwide for males, and the fifth most common cancer overall. Within the 27 countries of the European Union, prostate cancer has emerged as the most frequent cancer amongst men, increasing rapidly over the past two decades.

Standard pharmacological therapy, consisting of ablation of androgens, is initially efficient, but most treated patients progressively develop the disease again and eventually die of cancer. Consequently, many efforts are being made to identify novel targets and agents useful for the treatment of this disease.

In recent years, the immunotherapy, or the prevention or treatment of disease with substances that stimulate the immune response, has become an interesting alternative to traditional therapy for the treatment of cancer.

The immune system is a pivotal element in the defense of the organism against microbial infections as well as in the control and surveillance of malignant neoplasms. Immune cells scan tissues with the objective of removing newly malignant cells before they turn into fully formed tumors. Innate immune cells recognize the intruding pathogen and trigger appropriate immune response with the help of Toll-Like receptors (TLRs). They are expressed in sentinel cells such as macrophages and therefore they are responsible for the macrophage activation and control of parasitic infections.(Trine H. Mogensen, Clin. Microbiol Rev. 2009 Apr; 22(2): 240-273). Among all the Toll family receptors, TLR4 recognizes a wide array of ligands, including lipopolysaccharide (LPS) a cell wall component of Gram-negative bacteria, responsible for macrophage activation and signal transduction. In recent years there has been evidences indicating that mutations in TLR4 were associated with risk of prostate cancer (Dekai Zhang et al. Front Immunol. 2014; 5: 352).

In addition, a notable discovery has been the identification of an over-expressed protein in the surface of the prostate cancer cells, namely, prostate specific membrane antigen (PSMA).

The document of Francesco Peri et al.: J. Med. Chem. 2014, 57, 3612-3622, discloses that activated TLR4 induces intracellular signaling leading to activation of transcription factors that result in cytokine and chemokine production and initiation of inflammatory and immune responses. The authors review in this document the molecular mechanisms of TLR4 activation (agonism) or inhibition (antagonism) by small organic molecules of both natural and synthetic origin.

In the document of Hagen Cramer et al.: Nucleosides, Nucleotides, and Nucleic Acids, 26:1471-1477, 2007, the authors link a stabilized 2-5A analog to a ligand, which specifically recognizes prostate specific membrane antigen (PSMA), thereby directing 2-5A directly into prostate cancer cells. PSMA, like other cell surface receptors, undergoes internalization, thereby mediating the internalization of a putative ligand. This makes PSMA an ideal target for the treatment of cancer in particular prostate cancer.

With the aim to find alternative treatments to the traditional ones, the present invention is based in programming of Toll like Receptor 4 so that it signals when it detects a prostate antigen (PSMA). In particular it proposes a bifunctional compound that stimulate the body's own immune defenses to treat or prevent the prostate cancer.

### DESCRIPTION OF THE INVENTION

With the aim of finding an alternative treatment of prostate cancer to those known in the state of the art, the inventors of the present invention have focused immunological techniques that take advantage of the natural defense mechanisms of the body.

In particular, the inventors of the present invention have carried out the design of a bifunctional molecule that is, on one side, an agonist of the TLR4 and, on the other side, an important inhibitor of the PSMA.

As indicated above, the PSMA is a protein overexpressed on the surface of the prostate cancer cells and has been object of attention by the scientific community as a viable target to treat or prevent the cancer.

Toll-like receptors (TLRs) constitute a central constituent of the immune system as they are expressed on innate immune cells and help recognise the intruding pathogens. In particular, TLR4 recognises a wide array of ligands, including lipopolysaccharide (LPS), a cell wall component of Gram-negative bacteria, responsible for macrophage activation and signal transduction. Recent evidence associates mutations in TLR4 with increased risk for prostate cancer.

The compound of the present invention chemically stimulates the toll-like receptors (TLR4) and successfully matches PSMA, inducing an immune response. The rationale was to trick macrophages into thinking that the prostate cancer cell is a bacterium and kill it.

Therefore, a first aspect of the present invention relates to a compound of formula (I): 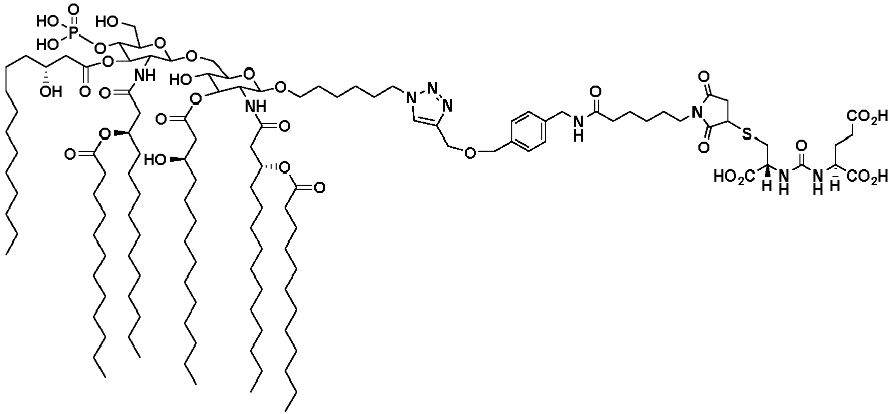 its pharmaceutically acceptable salts, tautomers and/or solvates.

The compound of formula (I) includes, on one side, a moiety that is an agonist of the TLR4 derived from monophosphoryl lipid A:

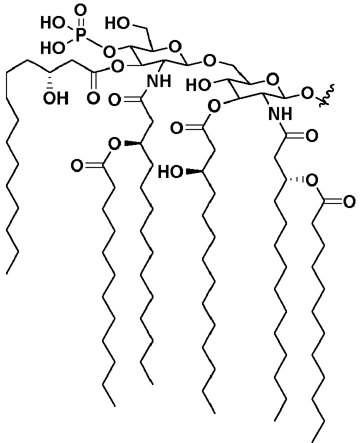

On the other side, the compound of formula (I) includes a moiety that is a urea-based PSMA inhibitor:

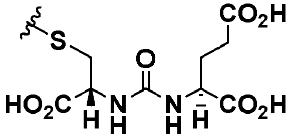

The PSMA inhibitor has been modified with a binder based on maleimide and aromatic moieties to allow the PSMA to bind and remain fixed on the surface of the prostate cancer cell. The binder moiety in the compound of formula (I) is:

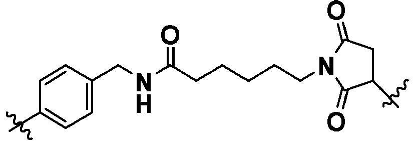

The agonist of TLR4 and the PSMA inhibitor modified with the binder are covalently linked in the compound of formula (I) by a linker based in triazole:

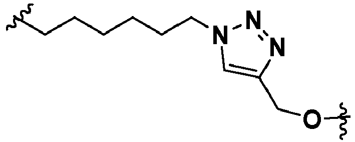

Therefore, a unique molecule (compound of formula (I)) includes two functionalities: an agonist of TLR4 and an inhibitor of PSMA, covalently linked by a linker moiety.

Unless otherwise stated, the compounds used in the invention are intended to include compounds that differ only in the presence of one or more isotopically enriched atoms. For example, compounds having the present structures except for the substitution of a hydrogen atom for a deuterium atom or a tritium atom, or the substitution of a carbon atom for a carbon atom enriched in ¹³C or ¹⁴C or a nitrogen atom enriched in ¹⁵N fall within the scope of this invention.

The term "pharmaceutically acceptable salts or solvates thereof" relates to salts or solvates which, on being administered to the recipient, are capable of providing a compound such as that described herein. The preparation of salts and derivatives can be carried out by methods known in the state of the art. Preferably, "pharmaceutically acceptable" relates to molecular entities and compositions that are physiologically tolerable and do not typically produce an allergic reaction or a similar unfavourable reaction, such as gastric upset, dizziness and similar side effects, when administered to a human. Preferably, the term "pharmaceutically acceptable" means approved by a regulatory agency of a federal or state government or collected in the US Pharmacopoeia or other generally recognised pharmacopeia for use in animals and, more particularly, in humans.

For example, pharmaceutically acceptable salts of the compound previously described herein are synthesised from the previously described compound containing a basic or acidic moiety by conventional chemical methods. In general, such salts are prepared, for example, by reacting the free acid or basic forms of the compound with a stoichiometric quantity of the appropriate base or acid in water or in an organic solvent or a mixture of both. Generally, non-aqueous media like ether, ethyl acetate, ethanol, isopropanol or acetonitrile are preferred. Examples of acid addition salts include addition salts of mineral acids such as, for example, hydrochloride, hydrobromide, hydroiodide, sulfate, nitrate, phosphate and addition salts of organic acids such as, for example, acetate, maleate, fumarate, citrate, oxalate, succinate, tartrate, malate, mandelate, methanesulfonate and p-toluenesulfonate. Examples of alkali addition salts include inorganic salts such as, for example, sodium, potassium, calcium, ammonium, magnesium, aluminium and lithium, and organic alkaline salts such as, for example, ethylenediamine, ethanolamine, N-dialkylenethanolamine, glucamine and basic amino acid salts.

The compounds of the invention may be in crystalline form, either as free compounds or as solvates (e.g.: hydrates), and it is understood that both forms fall within the scope of the present invention. Solvation methods are generally known in the state of the art. Suitable solvates are pharmaceutically acceptable solvates. In a particular embodiment, the solvate is a hydrate.

"Tautomers" are understood to be the two isomers that differ only in the position of a functional group because between the two forms there is a chemical balance in which a migration of a group or atom occurs.

The compound of formula (I) has been synthetized by a route based in "click chemistry", as detailed in examples of the present invention. Click chemistry, defined by Nobel laureate KB Sharpless and associates in 2001, is a class of biocompatible small molecule reactions commonly used in bioconjugation, allowing the joining of substrates of choice with specific biomolecules. Click chemistry is not a single specific reaction, but describes a way of generating products that follow examples in nature, which also generates substances by joining small modular units.

A second aspect of the present invention relates the compound of formula (I), as defined above, for use as a medicament.

Preferably, the medicament is a vaccine.

The term "vaccine", as used herein, relates to a composition that induces or enhances the protective immunity of an individual to a particular disease, such as cancer. In order to induce or enhance a protective immunity, the vaccine comprises the compound of formula (I) as defined above.

A third aspect of the present invention relates the compound of formula (I), as defined above, for use in immunotherapy. More preferably, for use in in immunotherapy for the treatment and/or prevention of prostate cancer.

In a preferred embodiment, the invention relates to the compound of formula (I) for use for the prevention of prostate cancer.

The present invention equally relates to a method for the prevention or treatment of prostate cancer which comprises administering, to a patient in need thereof, a therapeutically effective quantity of a compound of formula (I) as previously defined.

The term "therapeutically effective quantity" means the necessary quantity of a compound for the treatment or prevention of the disease, disorder or condition to be effective.

An additional aspect of the present invention relates to a pharmaceutical composition comprising the compound of formula (I) as defined above. The pharmaceutical composition comprises preferably at least one excipient, adjuvant and/or pharmaceutically acceptable vehicles.

The pharmaceutical composition is preferably a vaccine.

The pharmaceutical compositions can be administered by any suitable administration route, for example: oral, parenteral (subcutaneous, intraperitoneal, intravenous, intramuscular, etc.), rectal, etc.

The term "treatment or prevention" as used herein, unless otherwise indicated, relates to reversing, alleviating and inhibiting the progress of, or preventing the disorder or condition to which it applies in such terms, one or more symptoms of such disorder or condition.

The term "excipients, adjuvants and/or carriers" relates to molecular entities or substances through which the active ingredient is administered. Such pharmaceutical excipients, adjuvants or carriers can be sterile liquids, such as water and oils, including those of petroleum, animal, vegetable or synthetic origin, such as peanut oil, soybean oil, mineral oil, sesame oil and similar oils, excipients, disintegrating agents, humectants or dilutes. Suitable pharmaceutical excipients and carriers are widely known by the skilled person in the art.

In an embodiment of the invention, the pharmaceutical composition also comprises at least an active ingredient (active substance, therapeutic agent).

The term "active ingredient" refers to a compound with biological activity in the scope of the patent that, in small amounts may improve, promote, or complement the activity of compound of formula (I), such as other anticancer agent. Examples of active ingredients include lycopene, resveratrol, lignin, tannins, and indoles.

For its application in immunotherapy, the compound of formula (I) will preferably be in a pharmaceutically acceptable or substantially pure form, that is, the compound of formula (I) has a pharmaceutically acceptable level of purity excluding pharmaceutically acceptable excipients and not including material considered toxic at normal dosage levels. The purity levels for a compound of formula (I) are preferably above 50%, more preferably above 70%, more preferably above 90%. In a preferred embodiment, they are above 95%.

In general, the therapeutically effective amount of the compound of formula (I) to be administered will depend, among other factors, on the individual who is to be treated, the severity of the disease suffered by the individual, the selected form of administration, etc. For this reason, the doses mentioned in this invention must be considered solely as guides for the skilled person, who must adjust the doses according to the aforementioned variables. However, a compound of formula (I) may be administered one or more times a day, for example 1, 2, 3 or 4 times a day, in a typical total daily quantity comprised between 5 and 15 mg/kg body weight/day, preferably 10 mg/kg body mass/day.

The compound described in the present invention, its pharmaceutically acceptable, salts, tautomers and solvates and pharmaceutical compositions containing them may be used together with other additional drugs to provide a combined therapy. Said additional drugs may form part of the same pharmaceutical composition or, alternatively, may be provided as a separate composition for simultaneous administration or not with the pharmaceutical composition comprising a compound of formula (I), solvate or a pharmaceutically acceptable salt thereof.

As showed in the examples, the compound of the invention is stable in human serum. Stability of the product has been tested in human serum at 3 micromolar in concentration, showing its degradation after 18 hrs. Also binding studies have shown the efficacy of the compound for the treatment of prostate cancer.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

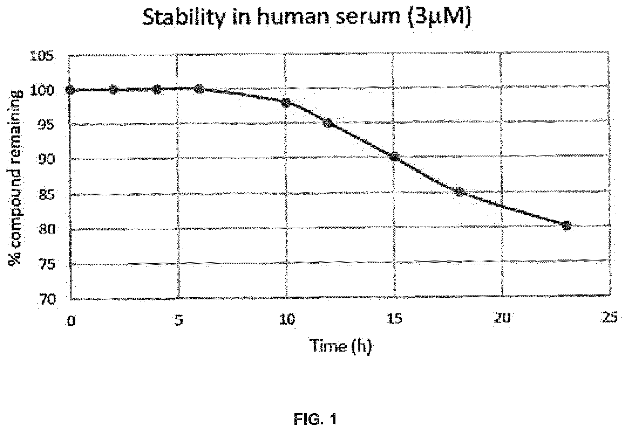

FIG. 1 Graph that shows the stability of the compound of the present invention in human serum.

### EXAMPLES

### Example 1: Synthesis of the compound of formula (I) of the present invention.

As indicated above, the compound of formula (I) has been synthetized by a route based in "click chemistry". So, the compound of formula (I) has been obtained by joining small modular units as described in the experimental procedure.

### Experimental procedure:

### Materials and Methods

**The starting materials:** dodecanaldehyde; Fmoc-Cys(MMt)-OH; (4-Hydroxymethylbenzyl)carbamic acid tert-butyl ester; glucosamine hydrochloride were obtained from Aldrich, Alfa Aesar, Maybridge and Novabiochem,, respectively and were used without any further purification.

**Nuclear Magnetic Resonance Spectroscopy (NMR)** experiments were recorded at Bruker DRX-600 spectrometer using chloroform-d (CDCl₃) as solvent. ¹H and ¹³C NMR chemical shifts (δ) are reported in ppm relative to residual CHCl₃ at 7.26 and 77.16 ppm respectively.

**Mass spectrometer:** HRMS spectra were measured using electrospray ionization (ESI). LC/MS (Hewlett-Packard1100 MSD) was performed using reversed-phase DiscoveryH18 column (3µm; 5 cm×4.6 mm) with a gradient elution (30-100% solvent B; solvent A = water, solvent B =acetonitrile) and detection at 254 nm. The MS (Agilent 100MSD) employed a scan range of 200-2000 MHz *m*/*z* positive mode; fragmentor = 100. Spray chamber conditions are as follows, dry gas flow of 12 L/min, nebulizer pressure of 50 psig, dry gas temp of 350°C, and a capillary voltage of 4000.

Firstly, the compound 6 was synthetized by following the route represented in Scheme 1. 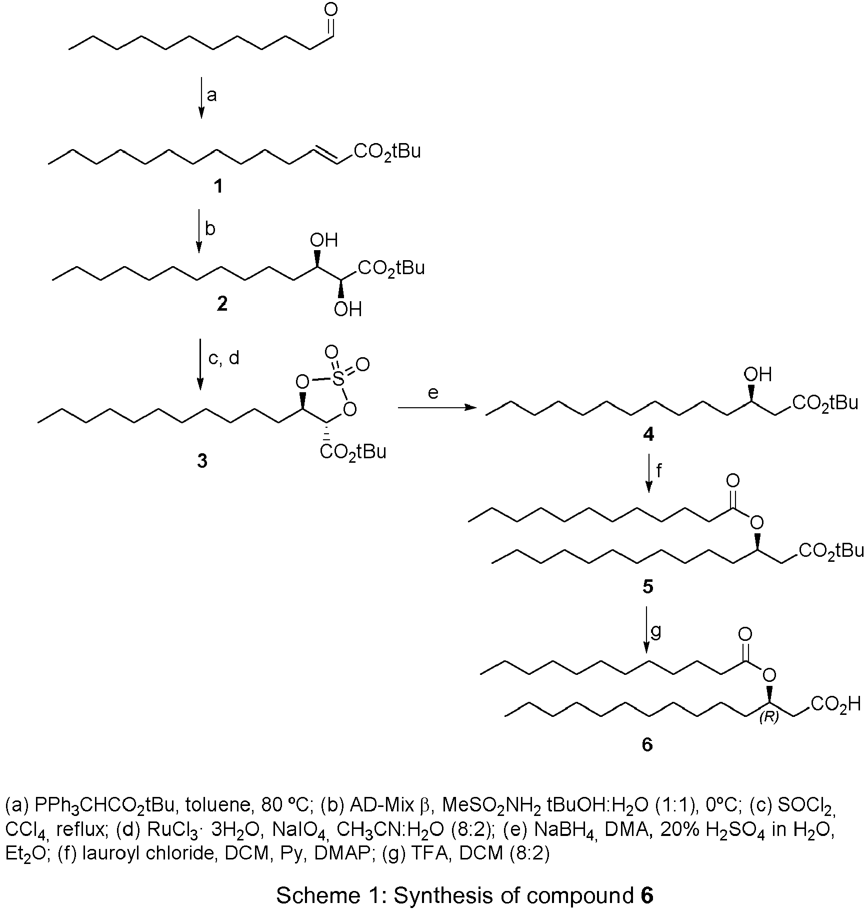
**Compound 1:** To a solution of dodecanaldehyde (5g, 27.2 mmol.) in dry toluene (250 mL) was added *tert*-butoxycarbonylmethylene triphenylphosphorane (15.5 g, 40.8 mmol.) and the mixture was heated up to 100 °C during 3 hours. Then, it is evaporated to dryness under reduced pressure and the crude reaction mixture was purified by silica gel column chromatogaphy using Hex:EtOAc (95:5) as eluent, to the desired ester as a colorless liquid (8 g, 95%).
**Compound 2:** AD-Mix β (40 g, 1.4 g/mmol of olefin) and methanesulfonamide (2.7 g, 28 mmol.) were dissolved in a mixture of *tert*-butanol: water (1:1) (v/v) (500 mL). Once dissolved, the solution was cooled down to 0 °C. Then, the reaction becomes a heterogeneous mixture and the olefin **1** (8 g, 28 mmol.) was added in one portion. The reaction was kept at 0 °C until TLC shows complete conversion (usually the reactions changes from an intense orange color to a pale-yellow color). Once the reaction is complete sodium sulfite (15 g.) is added and the reaction mixture is stirred vigorously for one hour. Afterwards, it was extracted with ethyl acetate (EtOAc), (3x 200 mL), and the organic fractions collected, dried with magnesium sulphate, filtered and evaporated under reduced pressure to afford an oil that was purified by silica gel column chromatogaphy using Hex:EtOAc (8.2) as eluent. The desired diol was obtained as a pale yellow oil (6.6 g, 75%).
**Compound 4:**
   Diol **2** (3g, 9.50 mmol.) was dissolved in carbon tetrachloride (20 mL) and thionyl chloride (0.85 mL, 11.4 mmol.) was added. The reaction mixture was refluxed for 30 min. Then, the solution was cooled down with an ice-water bath and diluted with acetonitrile (25 mL). RuCl₃·3H₂O (1.2 mg, 5.7 µmol.) and NaIO₄ (3 g, 14.25 mmol.) were added followed by water (30 mL). The resulting orange mixture was stirred at room temperature for 1 hour. The mixture was then diluted with ether (200 mL), and the two phases were separated. The organic layer was washed with water (20 mL), saturated aqueous NaHCO₃ (2 x 20 mL), and brine (20 mL). After drying over MgSO₄ the solution was filtered through a small pad of silica gel to remove the brown color. The filtrate was concentrated to afford the corresponding cyclic sulphate **3** which was used without any further purification. Cyclic sulphate **3** (3 g, 8 mmol.) was dissolved in dimethylacetamide (16 mL) and NaBH₄ (300 mg, 8 mmol.) was added. The reaction mixture was stirred at room temperature for 30 min. and then it was evaporated to dryness. The residue was stirred with 20% aqueous H₂SO₄ (40 mL) and ether (40 mL) for 12 hours. Then, the reaction was extracted with ether (2 x 100 mL) and the combined organic layers were dried over MgSO₄ and evaporated to dryness. After evaporation, the crude product was chromatogaphied by column chromatogaphy using Hex:EtOAc (9:1) to afford the corresponding alcohol **4** as a colorless oil. (4.5 g, 70% two-steps).
   ¹H-NMR (CDCl₃, 600 MHz): δ. ¹³C-NMR (CDCl3, 100 MHz): δ. HRMS (ESI-TOF high-acc): m/z[MNa+]calcd. for C₁₈H₃₆O₃:323.2556; found: 323.2555.
**Compound 5:**
   Alcohol **4** (1.3 g, 4.3 mmol.) was dissolved in dry dichloromethane (10 mL) and lauroyl chloride (1.2 mL, 5.2 mmol.), pyridine (0.42 mL, 5.2 mmol.) and dimethylaminopyridine (10 mg) were subsequently added. The reaction mixture was stirred at room temperature for 24 hours and then, it was evaporated to dryness. The crude product was purified by column chromatogaphy using Hex:EtOAc (95:5) to afford the ester as a white solid (1.9 g, 90%).
**Compound 6:**
   Ester **5** (2.2 g, 4.56 mmol.) was dissolved in a mixture of trifluoroacetic acid:dichloromethane (8:2) (8 mL of trifluoroacetic acid, 2mL of dichloromethane), and the mixture was stirred at room temperature for 2 hours. Then, it was evaporated to dryness under reduced pressure to afford the pure acid. (2 g, >99%)
   ¹H-NMR (CDCl₃, 600 MHz): δ 5.23 (quint, J = 4 Hz, 1H), 2.63 (dq, J = 4 Hz, 2H), 2.30 (t, J = 4 Hz, 2H), 1.63 (m, 5H), 1.34 (m, 34H), 0.91 (m, 6H). ¹³C-NMR (CDCl3, 100 MHz): δ 175.17, 172.82, 69.51, 38.35, 34.02, 33.52, 31.46, 29.17, 29.08, 29.03, 28.89, 28.82, 28.66, 24.65, 24.55, 22.23, 13.65. HRMS (ESI-TOF high-acc): m/z[MH+]calcd. for C₂₆H₅₆O₄:427.3782; found: 427.3783.
   Then, the compound 12 was synthetized by following the route represented in Scheme 2. 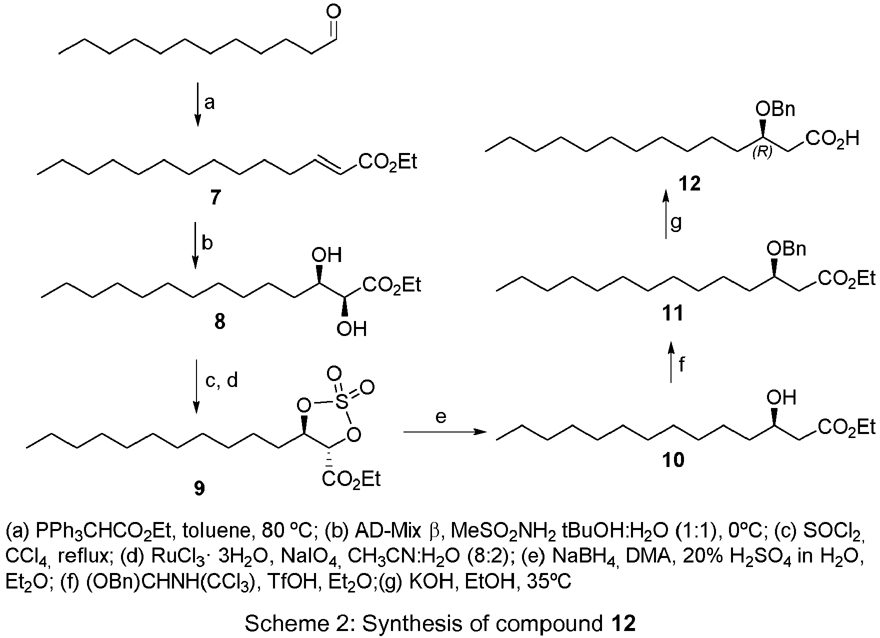
**Compound 7:** To a solution of dodecanaldehyde (5g, 27.2 mmol.) in dry toluene (250 mL) was added carbethoxymethylene triphenylphosphorane (14.2 g, 40.8 mmol.) and the mixture was heated up to 100 °C during 3 hours. Then, it is evaporated to dryness under reduced pressure and the crude reaction mixture was purified by silica gel column chromatogaphy using Hex:EtOAc (95:5) as eluent, to afford the desired ester as colorless liquid (6.5 g, 95%).
**Compound 8:** AD-Mix β (36 g, 1.4 g/mmol of olefin) and methanesulfonamide (2.5 g, 26 mmol.) were dissolved in a mixture of *tert*-butanol: water (1:1) (v/v) (500 mL). Once dissolved, the solution was cooled down to 0 °C. Then, the reaction becomes a heterogeneous mixture and the olefin **7** (6.5 g, 26 mmol.) was added in one portion. The reaction was kept at 0 °C until TLC shows complete conversion (usually the reactions changes from an intense orange color to a pale-yellow color). Once the reaction is complete sodium sulfite (15 g.) is added and the reaction mixture is stirred vigorously for one hour. Afterwards, it was extracted with ethyl acetate (EtOAc), (3x 200 mL), and the organic fractions collected, dried with magnesium sulphate, filtered and evaporated under reduced pressure to afford an oil that was purified by silica gel column chromatogaphy using Hex:EtOAc (8.2) as eluent. The desired diol was obtained as a pale yellow oil (5.8 g, 78%).
**Compound 10:**
   Diol 8 (3g, 10.4 mmol.) was dissolved in carbon tetrachloride (20 mL) and thionyl chloride (0.91 mL, 12.5 mmol.) was added. The reaction mixture was refluxed for 30 min. Then, the solution was cooled down with an ice-water bath and diluted with acetonitrile (25 mL). RuCl₃·3H₂O (1.3 mg, 6.2 µmol.) and NaIO₄ (3.4 g, 15.6 mmol.) were added followed by water (30 mL). The resulting orange mixture was stirred at room temperature for 1 hour. The mixture was then diluted with ether (200 mL), and the two phases were separated. The organic layer was washed with water (20 mL), saturated aqueous NaHCO₃ (2 x 20 mL), and brine (20 mL). After drying over MgSO₄ the solution was filtered through a small pad of silica gel to remove the brown color. The filtrate was concentrated to afford the corresponding cyclic sulphate **3** which was used without any further purification. Cyclic sulphate **9** (3 g, 8.6 mmol.) was dissolved in dimethylacetamide (16 mL) and NaBH₄ (325 mg, 8.6 mmol.) was added. The reaction mixture was stirred at room temperature for 30 min. and then it was evaporated to dryness. The residue was stirred with 20% aqueous H₂SO₄ (40 mL) and ether (40 mL) for 12 hours. Then, the reaction was extracted with ether (2 x 100 mL) and the combined organic layers were dried over MgSO₄ and evaporated to dryness. After evaporation, the crude product was chromatogaphied by column chromatogaphy using Hex:EtOAc (9:1) to afford the corresponding alcohol **10** as a colorless oil. (2.15 r, 76% two-steps).
**Compound 11:**
   Alcohol **10** (2 g, 7.4 mmol.) was dissolved in dry ether (30 mL) and benzyl 2,2,2-trichloroacetimidate (2.7 mL, 14.8 mmol.), was added followed by trifluoromethanesulfonic acid (6.5 µL, 0.74 mmol.) The reaction mixture was stirred at room temperature for 3 hours and then, it was evaporated to dryness. The crude product was purified by column chromatogaphy using Hex:EtOAc (95:5) to afford the O-benzyl protected alcohol **11** as a yellowish liquid (2 g, 75%).
**Compound 12:**
   Ester **11** (2 g, 5.5 mmol.) was dissolved in methanol (6 mL) followed by addition of potassium hydroxide (925 mg, 16.5 mmol.). The reaction mixture was heated up to 35 °C for 1 hour, and then cooled down to room temperature. The reaction mixture was extracted with water (50 mL) and ether (50 mL). The organic phase was separated and disregarded. The aqueous phase was acidified to pH=1 using 10% aqueous HCl solution and extracted with ether (50 mL). The organic phase was collected, dried over magnesium sulphate, and evaporated under vacuum to afford the pure acid **12** as a yellow liquid. (1.6 g, 88%)
   ¹H-NMR (CDCl₃, 600 MHz): δ 7.31 (m, 5H), 4.60 (s, 2H), 3.89 (brs, 1H), 2.65 (dd, J = 8 & 4 Hz, 1H), 2.58 (dd, J = 8 & 4 Hz, 1H), 1.70 (m, 1H), 1.61 (m, 1H), 1.35 (m, 19H), 0.91 (t, J = 4 Hz, 3H).. ¹³C-NMR (CDCl3, 100 MHz): δ 175.34, 137.52, 127.96, 127.41, 127.32, 75.30, 71.09, 38.84, 33.61, 31.46, 29.19, 29.17, 29.12, 29.09, 28.89, 24.65, 22.24, 13.67. HRMS (ESI-TOF high-acc): m/z[MH+]calcd. for C₂₁H₃₄O₃:335.2581; found: 335.2580.
   Then, the compound 25 was synthetized by following the route represented in Scheme 3. 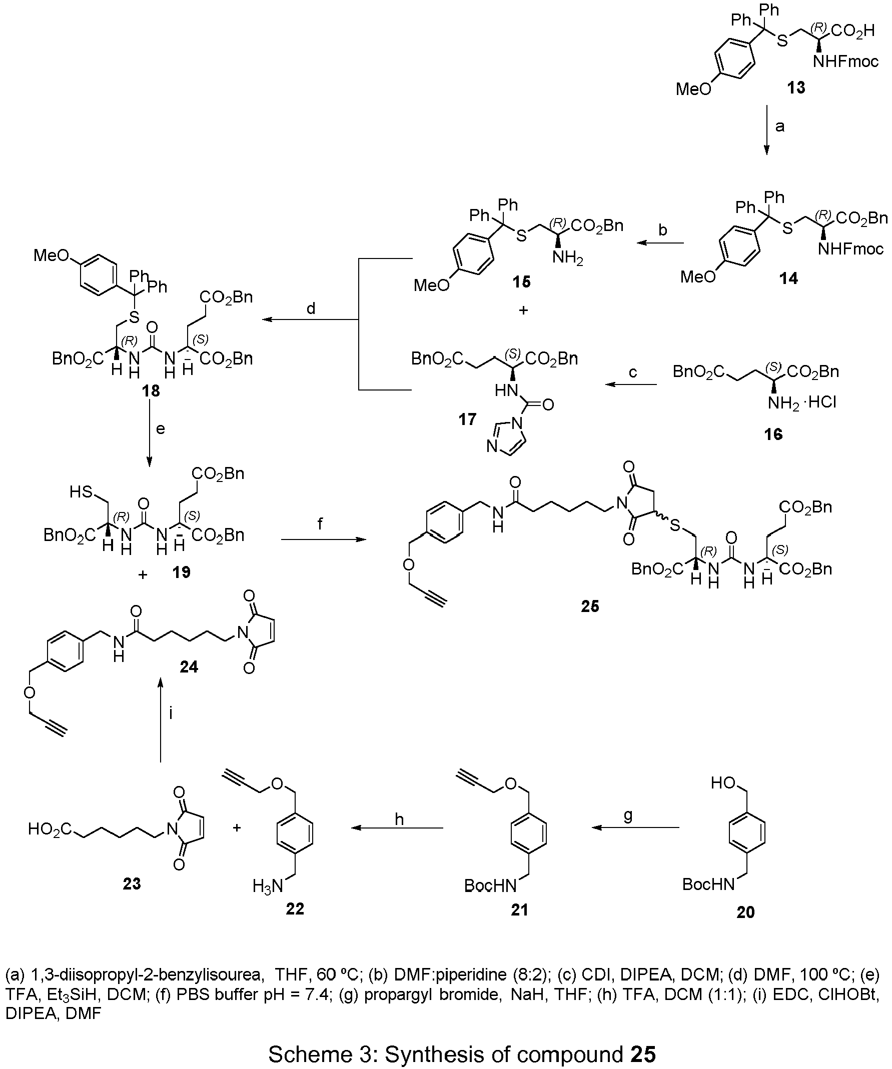
**Compound 14:**
   Fmoc-Cys(MMt)-OH **13** (3g, 4.9 mmol.) and 1,3-diisopropyl-2-benzylisourea (2.4 g, 12.2 mmol.) were dissolved in dry THF (120 mL) and heated at 60 °C overnight. Then, the reaction was extracted with EtOAc (100 mL) and 10% HCl aqueous solution (50 mL). The organic phase was dried over magnesium sulphate and evaporated to dryness under reduced pressure. The crude reaction mixture was purified by silica gel column chromatography using Hex:EtOAc (8:2) as eluent to afford Fmoc-Cys(MMt)-OBn **14** as a yellow solid (2.6 g, 75%).
**Compound 15:**
   Fmoc-Cys(MMt)-OBn **14** (200 mg, 0.298 mmol.) was dissolved in dry DMF (10 mL) and piperidine (3 µL, 0.03 mmol.) was added. The reaction is stirred at room temperature for 30 min. Then, it was evaporated to dryness under reduced pressure and purified by silica gel column chromatography using Hex:EtOAc (7:3) as eluent to afford H-Cys(MMt)-OBn **15** as a yellowish solid (100 mg, 69%).
**Compound 17:**
   H-Glu(OBn)-OBn·HCl **16** (100 mg, 0.275 mmol.) was dissolved in dry dichloromethane (10 mL) and N,N-diisopropylethylamine (0.2 mL, 1.2 mmol.) was added. The reaction was stirred at room temperature for 30 min, followed by addition of 1,1'-Carbonyldiimidazole (45 mg, 0.275 mmol.) and 4-(dimethylamino)pyridine (10 mg, 82 µmol.). The reaction was stirred at room temperature overnight. Then, it was washed subsequently with a NaHCO₃ saturated aqueous solution (2 x 20 mL) and brine (2 x 20 mL). The organic phase was dried over magnesium sulphate and purified by column chromatography using EtOAc as eluent to afford the urea **17** as a white solid (110 mg, 95%).
**Compound 18:**
   Urea **17** (87 mg, 0.207 mmol.) and H-Cys(MMt)-OBn **15** (100 mg, 0.207 mmol.) were dissolved in dry DMF (10 mL) in a sealed tube. The reaction was heated overnight at 100 °C. Then, it was evaporated to dryness under reduced pressure and the crude reaction mixture was purified by silica gel column chromatography using Hex:EtOAc (6:4) as eluent to afford the urea as a pale yellow oil (170 mg, 98%).
**Compound 19:**
   Urea **18** (170 mg, 0.203 mmol.) was dissolved in dry dichloromethane. To it, it was added triethylsilane (65 µL, 0.406 mmol.) followed by trifluoroacetic acid (23 µL, 0.365 mmol.). The reaction was stirred at room temperature until TLC shows complete conversion (usually within 3 hours). The reaction was evaporated to dryness under reduced pressure and purified by silica gel column chromatography using Hex:EtOAc (1:1) as eluent to afford **19** as a yellowish oil (110 mg, 97%).
   ¹H-NMR (CDCl₃, 600 MHz): δ 7.36 (s, 15 H), 6.00 (d, J = 6 Hz, 1H), 5.90 (d, J = 6 Hz, 1H), 5.26-5.11 (m, 6H), 4.88 (brs, 1H), 4.64 (brs, 1H), 2.98 (m, 2H), 2.45 (m, 2H), 2.23 (m, 1H), 2.03 (m, 1H), 1.31 (m, 1H). ¹³C-NMR (CDCl3, 100 MHz): δ 172.48, 172.36, 170.51, 156.26, 135.31, 134.66, 128.20, 128.17, 128.12, 128.10, 128.01, 127.93, 127.79, 127.75, 67.15, 66.99, 66.07, 54.02, 52.11, 29.83, 27.54, 27.36. HRMS (ESI-TOF high-acc): m/z[MH+]calcd. for C₃₀H₃₂N₂O₇S: 565.2003; found: 565.2002.
**Compound 21:**
   (4-Hydroxymethylbenzyl)carbamic acid tert-butyl ester **20** (500 mg, 2.1 mmol.) was dissolved in dry THF (40 mL) and sodium hydride 60% dispersion in mineral oil (126 mg, 3.15 mmol.) was added. After 30 minutes, propargyl bromide 80% wt in toluene (0.3 mL, 2.52 mmol.) was added and the reaction was allowed to react at room temperature overnight. Then, water was carefully added, and the reaction mixture was extracted with ether (50 mL) and NH₄Cl (20 mL) aqueous solution. The crude reaction mixture was purified by silica gel column chromatography using Hex:EtOAc (9:1) as eluent to afford the propargylated alcohol **21** as a yellow oil (320 mg, 55%).
**Compound 22:**
   Propargyl derivative **21** (320 mg, 1.16 mmol.) was dissolved in a mixture of dichloromethane and trifluoroacetic acid (1:1) (6 mL/each) and stirred at room temperature for 1 hour. Then, it was evaporated to dryness under reduced pressure to afford pure TFA salt **22.** (310 mg, 93%).
**Compound 24:**
   6-maleimidocaproic acid **23** (230 mg, 1.10 mmol.) was disolved in dry DMF (10 mL). To the reaction mixture were added EDC (210 mg, 1.10 mmol.) and CIHOBt (186 mg, 1.10 mmol.) and it was stirred at room temperature for 30 min. Then, amine 21 (320 mg, 1.10 mmol.) followed by N,N-diisopropylethylamine (0.2 mL, 1.10 mmol.) were added. The reaction was stirred at room temperature overnight, and extracted with EtOAc. The organic phase was dried over magnesium sulphate and chromatographied by silica gel column using Hex:EtOAc (2:8) as eluent to afford maleimide **24** as a white solid (275 mg, 68%).
   ¹H-NMR (CDCl₃, 600MHz): δ 7.34 (d, J = 8 Hz, 4H), 7.27 (d, J = 8 Hz, 4H), 6.68 (s, 4H), 5.83 (s, 2H), 4.61 (s, 4H), 4.44 (d, J = 6 Hz, 4H), 4.19 (d, J = 2 Hz, 4H), 3.52 (t, J = 7 Hz, 4H), 2.49 (t, J = 2 Hz, 2H), 2.22 (t, J = 7 Hz, 4H), 1.74-1.68 (m, 1H), 1.61 (q, J = 7 Hz, 4H), 1.37-1.26 (m, 4H). ¹³C-NMR (CDCl3, 100 MHz): δ 172.28, 170.38, 137.48, 136.19, 133.59, 128.05, 127.48, 79.10, 74.24, 70.71, 56.67, 42.92, 37.09, 35.98, 27.75, 25.82, 24.59. HRMS (ESI-TOF high-acc): m/z[MH+]calcd. for C₂₁H₂₄N₂O₄: 369.1809; found: 369.1806.
**Compound 25:**
   Maleimide **24** (75 mg, 0.20 mmol.) and cysteine **19** (110 mg, 0.20 mmol.) were dissolved in a minimum amount of DMF (1 mL). To it, phosphate buffer pH=7.4 was added (30 mL) and the reaction mixture was stirred at room temperature for 3 hours. Then, it was extracted with EtOAc. The organic layer was dried over magnesium sulphate and evaporate to dryness to afford pure **25** (180 mg, 97%).
   ¹H-NMR (CDCl₃, 600 MHz): Diastereomeric mixture δ 7.85 (t, J = 8 Hz, 2H), 7.55 (dt, J = 4 Hz, 1H), 7.41-7.30 (m, 19 H), 6.74 (m, 1H), 6.69 (m, 1H), 6.45 (m, 1H), 6.30 (d, J = 6 Hz, 1H), 5.22-5.07 (m, 7 H), 4.86 (m, 1H), 4.64 (s, 2H), 4.55 (m, 1H), 4.20 (s, 2H), 4.14 (m, 2H), 3.87-3.69 (m, 1H), 3.45 (m, 2H), 3.34-3.25 (m, 2H), 3.17 (m, 1H), 3.00 (m, 1H), 2.46 (brs, 1H), 2.40 (m, 2H), 2.18 (brs, 1H), 1.94 (m, 1H), 1.55 (m, 4H), 1.26 (m, 2H). ¹³C-NMR (CDCl3, 100 MHz): (Diastereomeric mixture) δ 177.21, 176.85, 174.30, 174.26, 172.83, 172.54, 172.20, 172.14, 170.90, 170.44, 168.87, 168.74, 167.31, 162.15, 156.71, 156.64, 141.12, 135.31, 134.88, 134.70, 132.36, 128.13, 128.12, 128.08, 128.02, 127.93, 127.91, 127.89, 127.76, 127.69, 127.60, 127.56, 127.39, 126.98, 126.96, 78.89, 74.55, 70.36, 66.69, 66.95, 66.67, 66.62, 65.96, 65.94, 57.04, 53.36, 52.50, 52.02, 52.00, 43.28, 40.05, 39.25, 38.71, 38.56, 37.91, 37.73, 36.30, 36.05, 35.59, 34.39, 34.29, 29.99, 29.89, 27.76, 27.60, 27.33, 27.02 HRMS (ESI-TOF high-acc): m/z[MH+]calcd. for C₅₁H₅₆N₄O₁₁S:933.3739; found: 933.3743.
   Then, the compound 36 was synthetized by following the route represented in Scheme 4. 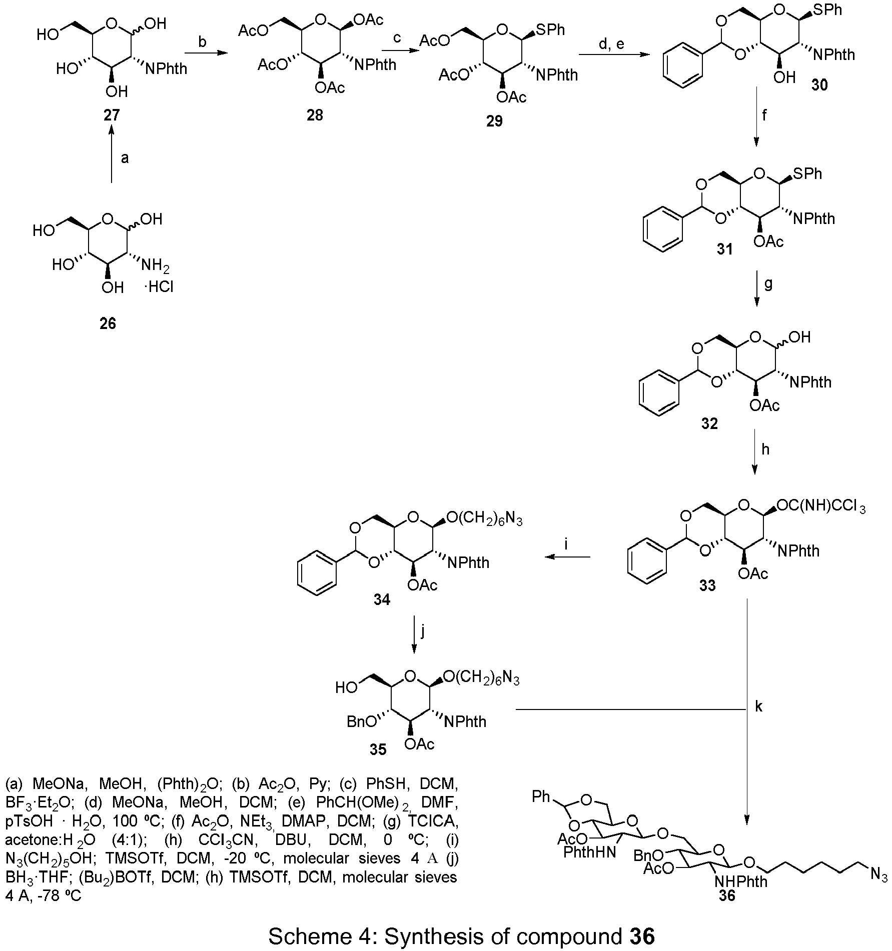
**Compound 27:**
   In a 500 mL round bottom flask it was placed 1.6 g of sodium in 50 mL of methanol. One the sodium is dissolved 10 g (46.5 mmol.) of glucosamine hydrochloride were added. The mixture was stirred for 30 min. and the precipitate formed was filtered. To the filtrate it was added phthalic anhydride (10.30 g, 69.75 mmol.) and it was stirred at room temperature until a white precipitate was formed. The precipitate was filtered and dried in the oven which gave us the N-phthalyl protected glucosamine as a white solid (14 g, 97%).
**Compound 28:**
   N-Pthalyl glucosamine **27** (14 g, 45.3 mmol.) is dissolved in a mixture of pyridine (100 mL) and acetic anhydride (100 mL), and it was heated at 80 °C overnight. Then, the reaction mixture was evaporated to dryness under reduced pressure and the crude reaction mixture was chromatographied with silica gel column using Hex:EtOAc (6:4) as eluent to afford derivative **28** as a yellowish syrup. (17 g, 78%).
**Compound 29:**
   5 g (10.48 mmol.) of derivative **28** were dissolved in dry dichloromethane. To it, thiophenol (1 mL, 10.48 mmol.) and BF₃·Et₂O (1.3 mL, 10.48 mmol.) were added. The reaction was stirred at room temperature overnight. Then, it was extracted with a NaHCO₃ saturated aqueous solution (2 x 20 mL) and the organic layer dried over magnesium sulphate and evaporated under vacuum. The crude reaction mixture was chromatographed with silica gel column chromatography using Hex: EtOAc (6:4) to afford thiol derivative 29 as a yellowish foam (5 g, 90%).
**Compound 30:**
   Thiol 29 (5g, 9.48 mmol.) was dissolved in dry dichloromethane. Then, sodium methoxide (1M in methanol freshly prepared, 1.2 mL) was added. The reaction was allowed to react at room temperature for 3 days and it was followed by TLC. When TLC showed complete conversion of the reaction, it was evaporated under vacuum to complete dryness to afford a white solid. The solid was dissolved in dry DMF (100 mL) and benzaldehyde dimethyl acetal (2.14 mL, 14.22 mmol.) followed by p-toluenesulfonic acid monohydrate (90 mg, 0.5 mmol.) were subsequently added. The reaction was heated at 70 °C under vacuum until TLC showed complete conversion of the reaction. Then, it was evaporated to dryness and chromatographed with silica gel using Hex:EtOAc (6:4) as eluent. The product was obtained as a yellow oil. (3 g, 65%).
**Compound 31:**
   Derivative 30 (3 g, 6.2 mmol.) was dissolved in dry dichloromethane (80 mL) and acetic anhydride (0.9 mL, 9.3 mmol.) followed by triethylamine (2.6 mL, 18.6 mmol.) were subsequently added. The reaction was stirred overnight. Afterwards it was extracted with a NaHCO₃ saturated aqueous solution (2 x 20 mL) and the organic layer was dried over magnesium sulphate and evaporated under vacuum. The crude reaction mixture was purified by silica gel column chromatography using Hex:EtOAc (6:4) as eluent to afford **31** as colorless oil (2.9 g, 87%).
**Compound 32:**
   Thiol **31** (1.3 g, 2.45 mmol.) was dissolved in a mixture of acetone:water (4:1) (64 mL acetone: 16 mL of water). To it, trichloroisocyanuric acid (570 mg, 2.45 mmol.) was added and the mixture was vigorously stirred at room temperature for 2 hours. Then, the mixture was extracted with dichloromethane (100 mL) and NaHCO₃ saturated aqueous solution (2 x 50 mL). The organic layer was dried over magnesium sulphate and evaporated under vacuum. The crude reaction mixture was purified by silica gel column chromatography using Hex:EtOAc (6:4) as eluent. The product came out as a white solid (880 mg, 82%).
**Compound 33:**
   Lactol **32** (880 mg, 2 mmol.) is dissolved in dry dichloromethane. The solution was cooled down to 0 °C and trichloroacetonitrile (2 mL, 20 mmol.) and DBU (6 µL, 0.04 mmol.) were added. The reaction was stirred at 0 °C until TLC showed complete conversion. Once the reaction was finished the mixture was evaporated to dryness and purified by silica gel column chromatography using Hex: EtOAc (6:4) as eluent, to afford the trichloroacetimidate as a brown foam (1 g, 86%).
**Compound 34:**
   Trichloroacetimidate **33** (3g, 5.14 mmol.) was dissolved in dry dichloromethane (200 mL) 6-azido-1-hexanol (735 mg, 5.14 mmol.) and molecular sieves 4 Å were added to the reaction mixture and stirred at room temperature for 30 minutes. Then, the reaction was cooled down to -20 °C and trimethylsilyl triflate (50 µL, 2.57 mmol.) was added. The reaction was allowed to reach to room temperature and followed by TLC. When TLC showed complete conversion, trimethylamine (0.7 mL, 5.14 mmol.) was added to quench the reaction the reaction. Then, it was filtered through a pad of Celite®, the filtrate evaporated to dryness and purified by silica gel column chromatography using Hex:EtOAc (7:3) as eluent, to afford the azide **34** as a yellowish oil (2.4 g, 82%).
**Compound 35:**
   Azide **34** (2.4 g, 4.2 mmol.) was dissolved in BH₃·THF (1M solution in THF) (50 mL) and cooled down to 0 °C. Then, trimethylsilyl triflate (0.8 mL, 6.3 mmol.) and the reaction was stirred at 0 °C until TLC showed complete conversion (usually within 4 hours). Once the reaction was completed it was quenched by addition of trimethylamine (2mL) and methanol (10 mL). The solution was concentrated to dryness under reduced pressure and the crude reaction mixture was purified by silica gel column chromatography using Hex:EtOAc (1:1) as eluent to afford the corresponding alcohol **35** as colorless oil. (1.6 g, 67%).
**Compound 36:**
   Trichloroacetimidate **33** (2.8 g, 4.83 mmol.) and azide 35 (2.7 g, 4.83 mmol.) were dissolved in dry dichloromethane with molecular sieves 4 Å. The solution was stirred at room temperature for 1 hour. Then, it was cooled down to -78 °C and trimethylsilyl triflate (17 µL, 0.1 mmol.) was added. The reaction was allowed to warm to room temperature and followed by TLC. When TLC showed complete conversion, trimethylamine (1 mL) was added in order to quench the reaction. The reaction mixture was filtered through a pad of Celite® and the filtrate evaporated to dryness under vacuum. The crude reaction mixture was purified by silica gel column chromatography using toluene:EtOAc (9:1) to afford disaccharide **36** as a sticky oil (4.8 g, 65%).
   Then, the compound 39 was synthetized by following the route represented in Scheme 5. 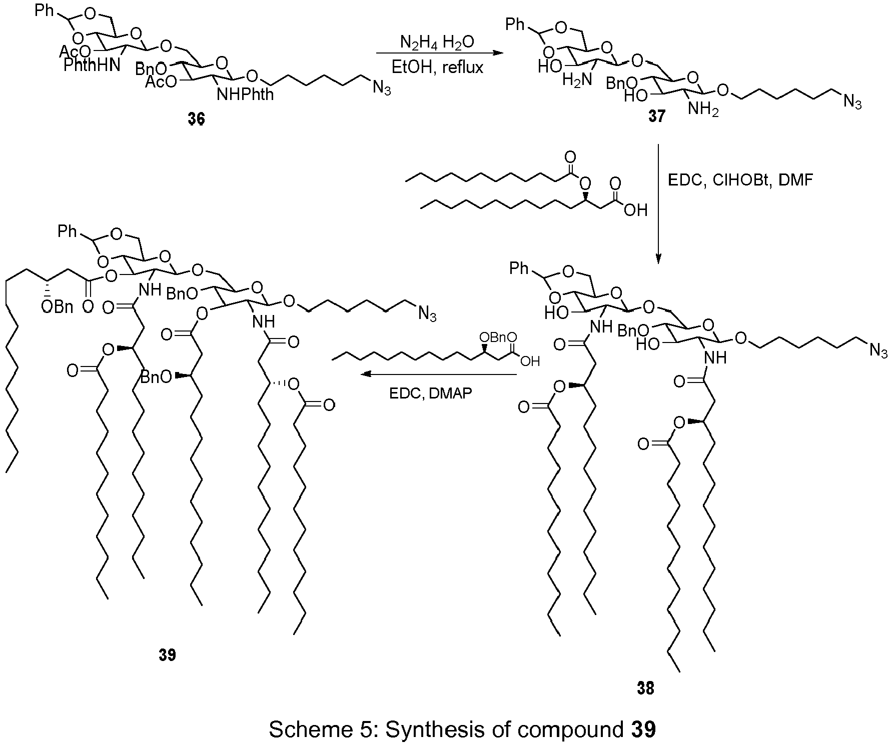
**Compound 37:**
   Disaccharide 36 (200 mg, 0.203 mmol.) was dissolved in dry ethanol (15 mL). Hydrazine hydrate (2.4 mL, 49.5 mmol.) was added and the reaction mixture was refluxed for 2 hours. Then, the solvent was evaporated under reduced pressure and the crude reaction mixture was purified by C₁₈ reversed phase silica gel column chromatography using acetonitrile:water (9:1) as eluent, to afford the corresponding disaccharide **37** as a white solid (100 mg, 76%).
**Compound 38:**
   Fatty acid **6** (20 mg, 0.047 mmol.) was dissolved in dry DMF (1 mL). EDC (13 mg, 0.07 mmol.) and CIHOBt (12 mg, 0.07 mmol.) were added and the reaction mixture was stirred at room temperature for 30 minutes. Afterwards, disaccharide **37** (15 mg, 0.023 mmol.) followed by DIPEA (1.6 µL, 0.093 mmol.) were added and the reaction stirred at room temperature overnight. Then, it was evaporated to dryness under reduced pressure and the residue dissolved in dichloromethane (20 mL) and washed with brine (10 mL). The organic layer was dried over magnesium sulphate, filtered and evaporated to dryness under reduced pressure to afford the crude reaction mixture, which was re-suspended in methanol allowing **38** to crash out as a white solid. The solid was filtered and dried to afford **38** (22 mg, 66%).
**Compound 39:**
   In a sealed tube, fatty acid **12** (10 mg, 0.030 mmol.) and disaccharide **38** (20 mg, 0.014 mmol.) were dissolved in dry 1,2-dichloroethane (2 mL). EDC (11 mg, 0.055 mmol.) was added followed by 4-dimethylaminopyridine (DMAP) (8 mg, 0.068 mmol.). The reaction was heated up to 100 °C overnight. The reaction mixture was washed with 1M HCl aqueous solution, and the organic layer was dried over magnesium sulphate and evaporated to dryness. Then, the crude reaction mixture was purified by preparative silica gel TLC using dichloromethane: acetone (12:1) as eluent, to afford diprotected disaccharide **39** (22 mg, 75%).
   ¹H-NMR (CDCl₃, 600MHz): δ 7.41-7.22 (m, 20H), 5.86 (d, J = 12 Hz, 1H), 5.81 (d, J = 12 Hz, 1H), 5.45 (s, 1H), 5.39 (t, J = 6 Hz, 1H), 5.32 (s, 1H), 5.21 (t, 6 Hz, 1H), 5.05 (m, 2H), 4.88 (d, J = 6 Hz, 1H), 4.61 (d, J = 12 Hz, 1H), 4.56-4.47 (m, 4H), 4.44 (d, J = 12 Hz, 1H), 4.35 (m, 1H), 4.02 (d, J = 6 Hz, 1H), 3.91-3.75 (m, 6 H), 3.68 (t, J = 12 Hz, 1H), 3.57 (brs, 2H), 3.50-3.46 (m, 2H), 3.25 (t, J = 6 Hz, 2H), 2.67 (ddd, J = 12 & 6 Hz, 1H), 2.59 (ddd, J = 12 & 6 Hz, 1H), 2.53-2.47 (m, 2H), 2.41-2.25 (m, 6 H), 2.20 (m, 1H), 1.62-0.89 (m, 146H). ¹³C-NMR (CDCl3, 100 MHz): δ 173.31, 173.10, 171.14, 170.76, 169.16, 168.97, 138.03, 137.98, 137.15, 136.41, 128.55, 127.98, 127.85, 127.83, 127.68, 127.42, 127.36, 127.25, 127.23, 127.09, 127.05, 100.94, 100.85, 100.22, 78.34, 75.37, 75.16, 75.01, 74.38, 73.79, 70.86, 70.70, 70.61, 70.39, 68.49, 68.15, 67.70, 66.01, 54.94, 53.66, 50.91, 41.37, 41.16, 39.12, 39.06, 34.06, 33.93, 33.72, 31.48, 29.25, 29.22, 29.20, 29.13, 29.11, 29.04, 28.99, 28.95, 28.92, 28.80, 28.32, 26.06, 25.12, 24.83, 24.80, 24.78, 24.75, 24.58, 24.56, 22.24, 13.66, 0.57. HRMS (ESI-TOF high-acc): m/z[MH+]calcd. for C₁₂₆H₂₀₅N₅O₁₉: 2093.5301; found: 2093.5296.
   Then, the compound 41 was synthetized by following the route represented in Scheme 6. 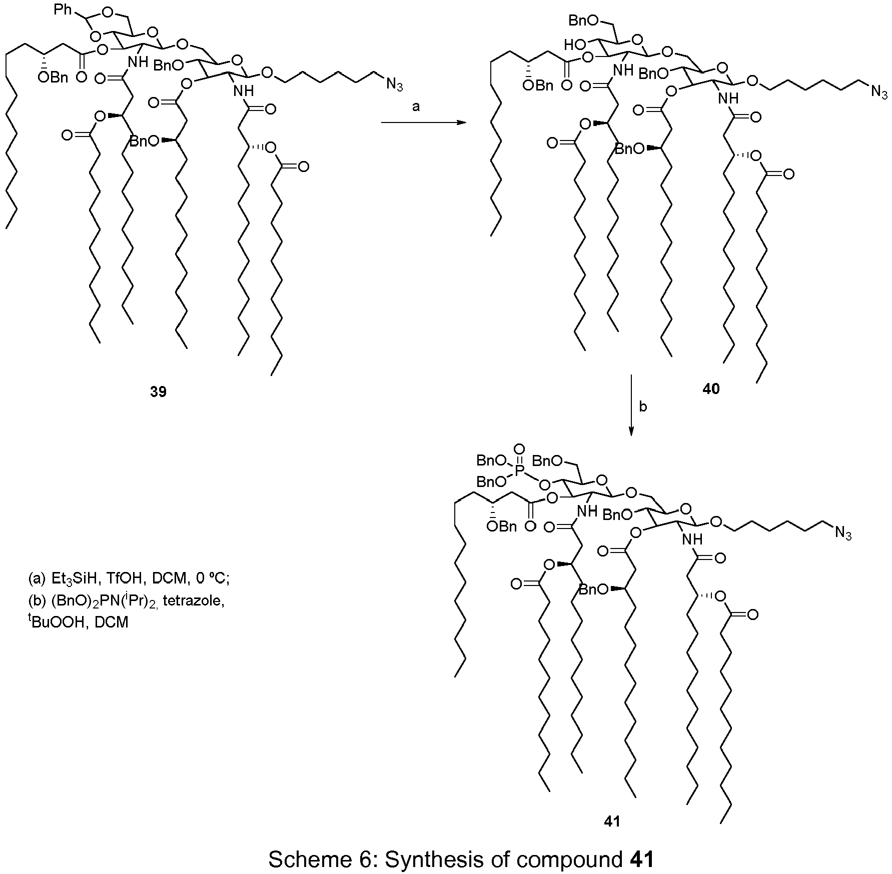
**Compound 40:**
   Disaccharide **39** (17 mg, 8 µmol.) was dissolved in dry dichloromethane (5 mL), and cooled down to 0 °C. Then, triethylsilane (37 µL, 16 µmol.) followed by trifluoromethanesulfonic acid (30 µL, 15 µmol.) were added, and the reaction mixture was stirred at 0 °C for 1 hour. The reaction was quenched by addition of trimethylamine (10 µL) and methanol (10 µL). The mixture was evaporated under reduced pressure. To the crude reaction mixture obtained, methanol was added and a white precipitate was formed. The precipitate was filtered and dried to afford the alcohol **40** as a white solid (10 mg, 58%).
   ¹H-NMR (CDCl₃, 600MHz): δ 7.35-7.22 (m, 20 H), 5.85 (d, J = 6 Hz, 1H), 5.77 (d, J = 6 Hz, 1H), 5.21 (t, J = 12 Hz, 1H), 5.05 (m, 3H), 4.70 (d, J = 6 Hz, 1H), 4.61-4.47 (m, 7H), 4.13 (s, 1H), 3.95 (d, J = 6 Hz, 1H), 3.89-3.74 (m, 7H), 3.65-3.57 (m, 4H), 3.45-3.38 (m, 3H), 3.28 (t, J = 6 Hz, 2H), 2.86 (brs, 1H), 2.65 (ddd, J = 18 & 12 Hz, 1H), 2.58-2.45 (m, 4H), 2.39-2.21 (m, 10H), 1.62 (m, 26H), 1.45-1.32 (m, 91H), 0.91-0.85 (m, 22H). ¹³C-NMR (CDCl3, 100 MHz): δ 173.12, 171.94, 171.13, 169.08, 137.99, 137.33, 137.23, 128.57, 127.99, 127.97, 127.84, 127.76, 127.52, 127.46, 127.42, 127.40, 127.29, 127.07, 125.69, 124.83, 107.15, 100.84, 100.77, 100.24, 75.51, 75.23, 75.04, 75.02, 74.36, 74.26, 73.79, 70.88, 70.61, 70.58, 70.50, 69.30, 68.71, 68.08, 62.02, 53.76, 53.73, 52.96, 50.92, 50.43, 41.35, 41.28, 39.17, 39.11, 34.05, 34.04, 33.70, 33.65, 33.41, 31.47, 29.25, 29.22, 29.19, 29.12, 29.10, 29.04, 29.01, 28.92, 28.79, 28.78, 28.31, 26.02, 25.04, 24.83, 24.81, 24.76, 24.68, 24.57, 24.55, 22.25, 13.25. HRMS (ESI-TOF high-acc): m/z[MH+]calcd. for C₁₂₆H₂₀₇N₅O₁₉: 2095.5457; found: 2095.5464.
**Compound 41:**
   Alcohol **40** (5 mg, 2.4 µmol.)was dissolved in a mixture (1:1) acetonitrile:dichloromethane (0.5 mL/each) under Ar atmosphere. Then, dibenzyl *N,N*-diisopropyl-phosphoramidite (2.4 µL, 7.2 µmol.) followed by 1*H*-tetrazole (27 µL of a solution 0.45 M in acetonitrile, 12 µmol.). The reaction mixture was stirred at room temperature overnight under Ar atmosphere. Then, tert-butylhydroperoxide (4.4 µL, 24 µmol.) of a 5.5 M solution in decane was added and the reaction was stirred at room temperature for 1 hour. The reaction mixture was evaporated under reduced pressure and purified by silica gel preparative TLC using dichloromethane: methanol (30:1) as eluent to afford **41** as a sticky oil. (3 mg, 53%).
   HRMS (ESI-TOF high-acc): m/z[MH+]calcd. for C₁₄₀H₂₂₀N₅O₂₂P: 2355.606; found: 2355.6061.
   Next, the compound 42 was synthetized by following the route represented in Scheme 7. 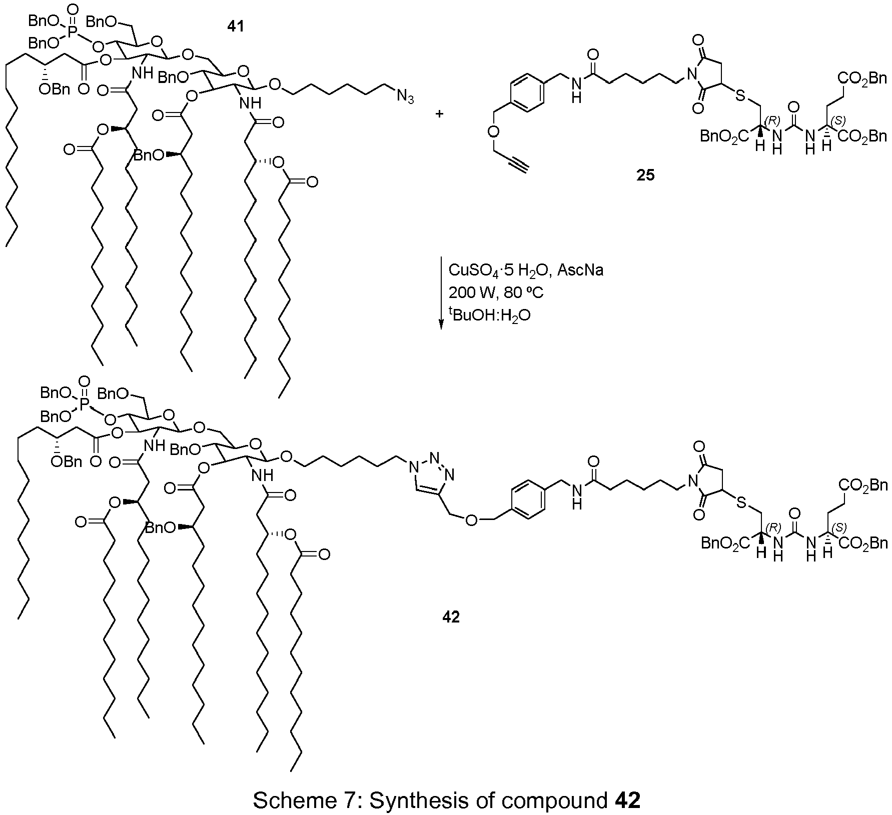
**Compound 42:**
   In a microwave tube **41** (3 mg, 1.3 µmol.) and **25** (1.2 mg, 1.3 µmol.) were suspended in a mixture of tert-butanol: water (1:1) (0.5 mL/each). Then, CuSO₄·5H₂O (16 µg, 0.065 µmol.) and (+)-*L*-Sodium Ascorbate (51 µg, 0.26 µmol.) were added and the reaction mixture was submitted to microwave conditions (80 °C, 200 W) for 90 minutes. The precipitate formed was centrifuged and dried to afford **42** (2.2 mg, 54%).
   HRMS (ESI-TOF high-acc): m/z[MH+]calcd. for C₁₉₁H₂₇₆N₉O₃₃SP: 3287.036; found: 3287.0362.
   Finally, the compound 43 (compound of formula (I) of the present invention) was synthetized by following the route represented in Scheme 8. 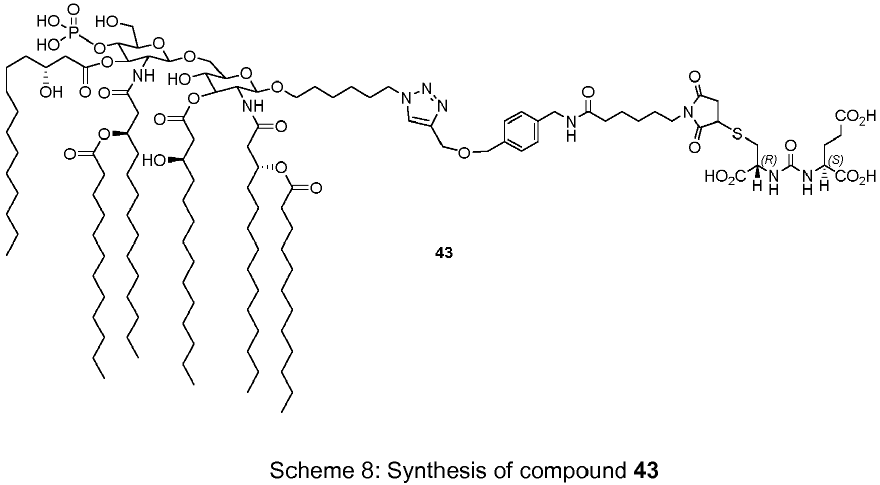
**Compound 43:**
   **42** (2.2 mg, 0.67 µmol.) was dissolved in a mixture (1:1) of dichloromethane and methanol (0.5 mL/each). To it, 10% palladium on carbon was added (catalytic amounts). The mixture was stirred under a hydrogen atmosphere at room temperature for 24 h. The Pd/C was then removed by filtration through a Teflon® filter, and the filtrate was concentrated to give a slurry which was purified by HPLC-MS using a VYDAC 218TP C₁₈- reversed phase silica column (250 mm X 22 mm, 10-15 µm) with acetonitrile (containing 0.1% trifluoroacetic acid) : water (3:7) as eluent at a flow rate 10mL/min to afford **43**. (1 mg, 60%).
   HRMS (ESI-TOF high-acc): m/z[MH+]calcd. for C₁₂₈H₂₂₂N₉O₃₃SP: 2477.550; found: 2477.5503.

### Example 2: Stability assays of the compound of formula (i) of the present invention

A reversed-phase HPLC (RP-HPLC) method, involving a gradient mobile phase system of water (solvent A) and acetonitrile (solvent B) containing 0.1% trifluoroacetic acid with 1 mL/min flow rate, was used to monitor the extent of the reaction. Compound **43** (10 mM solution in DMSO) was spiked into 0.9 mL fresh human serum and incubated at 37°C. Samples were collected at times indicated in the graph (0, 2, 4, 6, 10, 12, 15, 18, and 23 hrs.) and the sample was then injected onto a LC-MS with a C₁₈-reversed phase silica gel column, without further sample clean up or manipulation. The *in vitro* metabolic degradation was analyzed. The results are shown in the figure 1.

### Example 3: Cell inhibition assay and Relative binding studies

### Cell inhibition assay

A NAAG peptidase inhibition assay was undertaken to determine the IC₅₀ value for **43** and thus its inhibitory capacity for PSMA. The concentration of **43** was varied from 1 nM to 100 nM against a fixed amount of NAAG (4 µM) and a trace amount of [³H] NAAG. The NAAG peptidase (PSMA) was prepared from lysed PSMA-transfected CHO cells, purchased from ATCC (American Type Culture Collection). The percent enzymatic cleavage product, [³H] glutamate, was measured by scintillation counting and plotted against the logarithmic concentration of **43**. Linear regression of the resulting data was solved for 50% [³H] glutamate (50% inhibition) and resulted in an IC₅₀ value of 15 nM for **43**.

### Binding affinity of 43

The binding affinity of **43** to PSMA recombinant protein was measured using FET (Field effect transistor) techniques

### Protein expression and purification.

His-tagged PSMA (human) was purchased from Sinobiological, and stored at -20 °C following the manufacturer's protocol.

### FET chip functionalization and regeneration.

FET experiments (for measuring relative binding studies) were conducted on an Agile R100 (Nanomedical Diagnostics) at ambient temperature. Chips were functionalized with Ni-NTA according to manufacturer protocols. Surface-bound Ni-NTA sites were then bound to His-tagged protein by 15 min incubation with ∼100 nM protein in 50 mM MES (pH 6.0). Ni-NTA functionalized chips were regenerated via treatment with 250 mM imidazole for 30 mins, followed by extensive washing in MES, then reintroduction of His-tagged protein.

**Relative binding studies.** Test compound **43** was dissolved to desired concentration (10 µM) in MES (pH 6.0) buffer with 3% DMSO. Protein-bound chips were initialized and rinsed in MES buffer with 3% DMSO. Compound **43** was analyzed in a single run, and the R_{eq} determined. The affinity of **43** was determined by finding the ½ Rₘₐₓ for a concentration curve. The R_{eq} values were plotted against the concentration. A logarithmic line fit for this data was determined by Microsoft Excel, and the concentration of **43** for ½ Rₘₐₓ calculated from the equation for that line, giving an affinity of K_{D} = 8 nM ± 2.6 nM.

## Claims

1. Compound of formula (I): 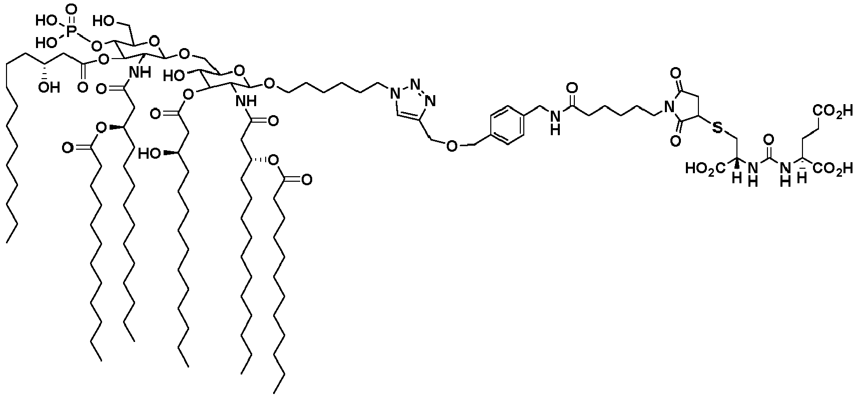 its pharmaceutically acceptable salts, tautomers and/or solvates.

2. Compound of formula (I), according to claim 1, for use as a medicament.

3. Compound of formula (I) for use, according to claim 2, wherein the medicament is a vaccine.

4. Compound of formula (I), according to claim 1, for use in immunotherapy.

5. Compound of formula (I) for use, according to claim 4, in the treatment and/or prevention of prostate cancer.

6. Pharmaceutical composition comprising the compound of formula (I) as defined in claim 1.

7. Pharmaceutical composition, according to claim 6, wherein the composition is a vaccine.

8. Pharmaceutical composition, according to claim 6 or 7, further comprising at least an active ingredient.
